# EUROPEAN PATENT APPLICATION

(11) **EP 3 922 224 A1**
(43) Date of publication of application: **15.12.2021**
(21) Application number: 19914479.1
(22) Date of filing: 22.11.2019
(51) Int. Cl.: A61F 7/00, A41D 13/005

(54) **CRYOTHERAPY GARMENT**

(30) Priority: 04.02.2019 ES 201930177 U
(71) Applicant: Leon Cid, Joaquin, 08301 Mataro (Barcelona) (ES)
(72) Inventor: Leon Cid, Joaquin, 08301 Mataro (Barcelona) (ES)
(74) Representative: Durán-Corretjer, S.L.P.
(86) International application number: PCT/ES2019/070800
(87) International publication number: WO 2020/161368

(57) **Abstract**

The invention relates to a cryotherapy garment comprising multiple receptacles for containing a cold-accumulating material, each receptacle being formed by a closed pleat of fabric of the garment and having two end openings for inserting a packet of cold-accumulating material.

## Description

The present invention discloses a garment useful for applying cold, applicable to the recovery after sports practice.

Cryotherapy is known as the application of any agent that lowers the temperature of the body's tissues for a therapeutic purpose.

In general, cold may be applied locally, by means of ice packs, instant cold packs, cold sprays, etc., or generally, by means of ice baths, cryosaunas or the like.

The application of cold to the body's tissues and the reduction of their temperature that this entails has a series of effects on these tissues:
- Antalgic or analgesic effect. Cold reduces sensitivity, producing local sedation.
- Vasoconstriction. The cold causes the blood vessels to contract, thus reducing the blood supply in the area.
- Anti-inflammatory. The cold, applied correctly, reduces the inflammation of muscles, tendons and ligaments caused by an injury or by excessive physical activity.
- Muscle relaxation. Cold reduces spasticity.

The use of cryotherapy in sports is known for its wide benefits both at a preventive and a curative level. At a preventive level, among other benefits, the application of cold favours muscle recovery and reduces fatigue. At the curative level, among other benefits, the application of cold helps reduce the inflammation associated with a large number of injuries, such as periostitis, tendonitis, bursitis, etc.

Despite the benefits that cryotherapy brings to sports practice, its use is not widely extended in part due to the difficulties that its application entails. With the exception of elite athletes, few athletes have ice baths or cold-water baths, cryosaunas, etc. at their disposal. This means that most athletes who use cryotherapy both preventively and curatively do so through the use of ice packs or "cold packs", the latter being understood as bags or packages that contain a chemical substance with a high calorific capacity and a high latent heat of fusion. Due to their nature, especially in terms of size, the application of cold is reduced to a relatively specific area of the body, which makes it necessary to use multiple ice packs or "cold packs" to treat a relatively large area, for example, the legs. Said multiple use entails associated problems, especially with regard to fixing them on a certain part of the body. In addition to the above, said multiple use entails problems in terms of the uniformity of the applied cold.

Document ES 1147037 U solves some of the aforementioned problems by disclosing a pair of cooling recovery tights that contain inside one or more bag-type compartments where a viscous gel capable of maintaining low temperatures without solidifying is stored. Said recovery tights additionally comprise an inner lining to avoid cold burns. The main drawback of this embodiment is that all the compartment designs shown result in an extremely expensive manufacturing cost for the item. On the other hand, in the tights disclosed by document ES 1147037 U, the gel may be removable or permanent, that is, it cannot be removed from the tights. In the embodiment where the gel cannot be removed, said tights comprise a single gel-filled pocket. The main drawback of this embodiment is that it is necessary to make a gel bag anatomically shaped. The creation of an anatomically shaped gel bag, as well as its subsequent encasing in the tights, is an expensive and complex process, which would entail that said tights be sold at a high price, thus considerably reducing the chances of commercial success. In addition, this embodiment would also be negatively impacted by its high weight, with the reduction in comfort of use that this entails. In the embodiment in which the gel can be extracted to be cooled in a freezer or the like to later be reintroduced into the tights to be used by the wearer, said tights comprise a plurality of pockets in which a plurality of gel sticks is inserted. Each pocket has a respective Velcro^{®} type closure. Among the disadvantages of this type of tights, removing and putting the gel sticks in their respective pockets can be quite cumbersome. In addition, the problem of this type of embodiment is that it requires the use of multiple gel bars of different lengths if a good anatomical fit is desired, which increases the stock of gel bars necessary for mass production of this type of tights, and therefore, increases the sale price of the same, thus reducing their chances of commercial success.

It is an object of the present invention to present a product that facilitates the use of cryotherapy, especially in the sports field, solving the aforementioned problems of the current prior art.

To that end, the present invention discloses a garment for cryotherapy comprising several receptacles to house a cold-accumulating material, in which said receptacles are each formed by a closed fold of fabric of the garment, each of said receptacles presenting two end openings for the introduction of a container of cold-accumulating material.

The embodiment using closed folds allows receptacles to be made which are suitable for receiving the container with the material during the same manufacturing process of the garment. The presence of two openings facilitates the simple introduction of containers of cold-accumulating material. For example, one opening has sufficient dimensions to allow the entry of a container that fills the receptacle, while the other opening may have different dimensions, for example smaller, to facilitate the passage through it of utensils to introduce the containers of cold-accumulating material.

Preferably, the receptacles will have an elongated shape. A particularly preferred shape for the receptacles object of the present invention is toric or having a torus section. Toric or torus section refers to a receptacle formed by a fold that has a substantially circular and elongated cross section, developing along the front, side and rear parts of the garment.

Preferably too, said fold or folds are longitudinally closed along one or two lines of closure. Advantageously, said closure is made by weaving techniques. In this way, the receptacles can be made simultaneously with the garment, for example, on a circular type knitting machine.

In a further inventive aspect of the present invention, the garment has containers of cold-accumulating material formed by concatenated malleable plastic capsules capable of being introduced into the receptacles through one of the aforementioned end openings of a respective receptacle. More preferably, the garment may be marketed as having a container of cold-accumulating material composed of several concatenated capsules arranged in a plurality of receptacles, and, even more preferably, in each of said receptacles.

The fact that the cold-accumulating material is packaged in concatenated malleable plastic capsules eliminates the need to have a large stock of gel bars of different sizes, as occurs in the prior art, since the arrangement in concatenated capsules makes it possible to obtain strips of cold-accumulating material of different lengths, depending on the production needs, from a very long single strip of said material. To do this, it is only necessary to cut a certain number of capsules, said number being that which is necessary to obtain a strip of cold-accumulating material of equal length to that of the receptacle intended to house it, thus obtaining a strip of cold-accumulating material ready to be inserted into the garment. If the method involved starting from a single continuous strip of cold-accumulating material, the result would not be the same, since, after cutting the strip of material, it would be necessary to close the open end, that is, the cut end. This does not happen in the present invention since the cut is made through a joining zone between capsules, thus obtaining a strip of cold-accumulating material the length of which is determined by the number of capsules it contains.

The new container format of cold-accumulating material according to the present invention facilitates not only cutting, but also the introduction of the container in elongated receptacles and with the preferred shapes indicated. In particular, using a reverse clamp that is inserted through one of the openings and stretches the material container, said container is inserted through the other opening of the receptacle. Additionally, the division into capsules facilitates the adaptation of the container to the shape of the body, since the joint area between capsules is more flexible than the capsules, facilitating the distribution of parallel and/or serpentine-shaped horizontal container lines.

Preferably, the concatenated capsules are spaced at regular intervals by pinching together. In a particularly preferred embodiment, the capsules are joined together by 1-centimetre pinching zones arranged every 4 centimetres.

Another advantage of the present invention is that it makes it possible to make compressive garments that combine pressure with cold. In this way, it is possible to combine the benefits of cryotherapy with the benefits of using compression garments, both differential and uniform. For this, preferably, the garment fabric comprises an elastomer. Additionally, the elasticity allows a firm grip on the cold-accumulating material container.

In certain embodiments, the longitudinal ends of said receptacle may be joined to or interlaced with each other. Also in certain embodiments, each of the longitudinal ends of said receptacle may be interlaced with or joined to the garment.

Advantageously, said receptacle may be arranged substantially perpendicular to the longitudinal axis of its respective extremity, that is, it may be arranged perpendicular to the longitudinal axis of the respective arm or leg covered by said garment. In embodiments where the garment also covers the trunk of the wearer, said receptacle may also be arranged substantially perpendicular to the longitudinal axis of the trunk.

Preferably, said receptacle and its respective cold-accumulating material may have a substantially annular or elliptical shape. Alternatively, said receptacle and its respective cold-accumulating material may have a substantially helical shape.

Advantageously, the garment object of the present invention may comprise a plurality of receptacles with their respective cold-accumulating material for each extremity covered by the garment, that is, the garment has a plurality of receptacles with their respective cold-accumulating material for each arm and/or leg which said garment covers. In the case of embodiments that cover the trunk of the wearer of the garment, said garment may also have multiple receptacles with their respective cold-accumulating material for said trunk.

Preferably, the spacing between the various receptacles with their respective cold-accumulating material may be constant, that is, the receptacles and their respective cold-accumulating material are evenly spaced from each other. Alternatively, the spacing between the different receptacles with their respective cold-accumulating material may be variable, that is, the spacing between the different receptacles with their respective cold-accumulating material varies throughout the garment. Said variation in the spacing between the different receptacles may be due to aesthetic reasons or functional reasons, that is, depending on the desired effect, part of the body to be treated, etc., or a combination of both.

Although the materials mentioned above are of preferred use for the manufacture of cryotherapy garments according to the present invention, it is also possible to manufacture them with other materials or fabrics.

Advantageously, said cold storage material may be a gel.

In the present document, a cold-accumulating material is understood to be any material with a high calorific capacity and/or a high latent heat of fusion. Preferably said cold storage material is of the eutectic type.

In a particularly advantageous embodiment, the garment for the cryotherapy object of the present invention may be tights, that is, a garment intended to cover the lower extremities of the wearer. Said tights may be long, usually covering approximately down to the ankle, or short, covering down to above the wearer's knee. Preferably, the receptacles cover from the iliac crest to the ankles, with the exception of areas corresponding to the inside of the legs, the genital area and the intergluteal cleft.

In one embodiment, the garment object of the present invention may cover the wearer's trunk by having receptacles in the trunk area. Thus, for example, the garment for cryotherapy object of the present invention may be a T-shirt, that is, a garment intended to cover the trunk. Said T-shirt may be a tank top, that is, sleeveless, short-sleeved or long-sleeved.

In one embodiment, the garment for cryotherapy object of the present invention may be an arm sleeve, that is, a garment intended to cover only the arms of the wearer, either totally or partially.

In one embodiment, the garment for cryotherapy object of the present invention may be a calf sleeve, that is, a garment intended to cover the calf of the wearer.

It should be understood that the above list of types of garments is not exhaustive and that the garment object of the present invention may, among others, take the form of combinations thereof, such as, for example, a jumpsuit.

Although the present invention was described using a cold-accumulating material, it should be understood that it may also be implemented by replacing the cold-accumulating material with an instantaneous cold generator, that is, by a container containing several elements that, after mixing, generate an endothermic reaction.

For a better understanding, several representative drawings of an embodiment of a garment for cryotherapy according to the present invention are attached, by way of explanatory but not limiting example.
- Figure 1 shows a front elevation view of an embodiment of a garment for cryotherapy according to the present invention.
- Figure 2 shows a rear elevation view of an embodiment of a garment for cryotherapy according to the present invention.
- Figure 3 schematically shows an embodiment of a cold-accumulating material according to the present invention.
- Figure 4 schematically shows a cross section of the receptacles designed to house a cold-accumulating material of an embodiment of a garment for cryotherapy according to the present invention.
- Figure 5 schematically shows a cross section of said receptacles housing their respective cold-accumulating material of an embodiment of a garment for cryotherapy according to the present invention.
- Figure 6 schematically shows the introduction of the cold-accumulating material into its respective receptacle.

In the figures, the same or equivalent elements were identified with identical numerals.

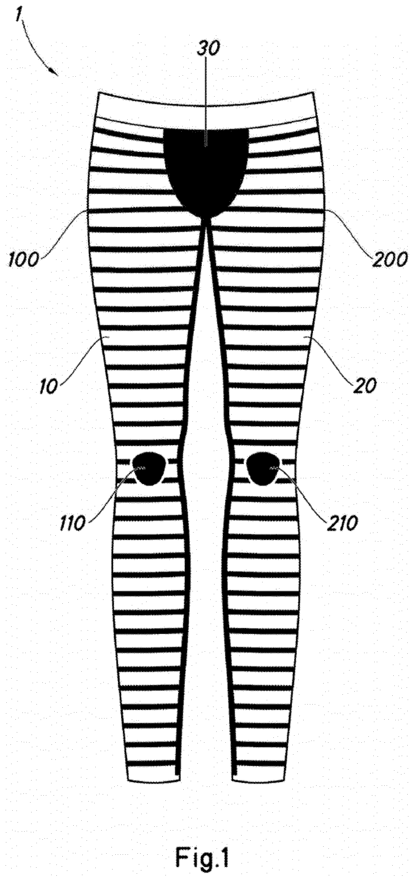

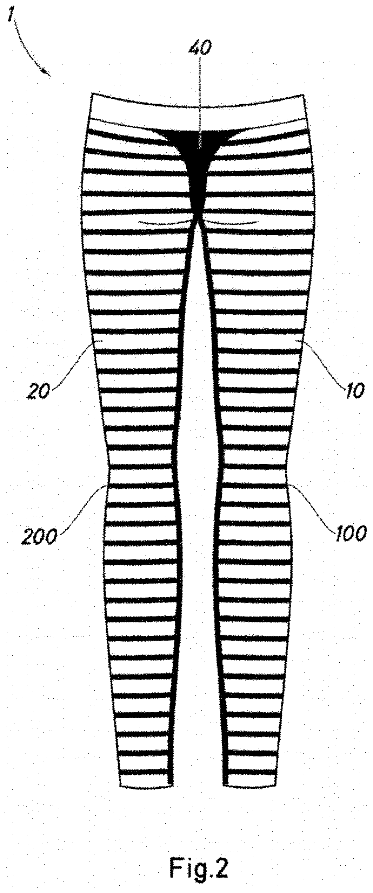

Figures 1 and 2 show, in a front elevation view and a rear elevation view, respectively, an embodiment of a garment 1 for cryotherapy according to the present invention. As can be seen, in the embodiment shown, the garment 1 is in the form of long tights, that is, of tights that cover down to approximately the ankles of the wearer thereof. In other embodiments, the tights may be short, that is, covering down to above the knee, or capri type, that is, covering down to about the middle of the calves. The trouser legs of the example, including receptacles, may be made on a circular-type machine.

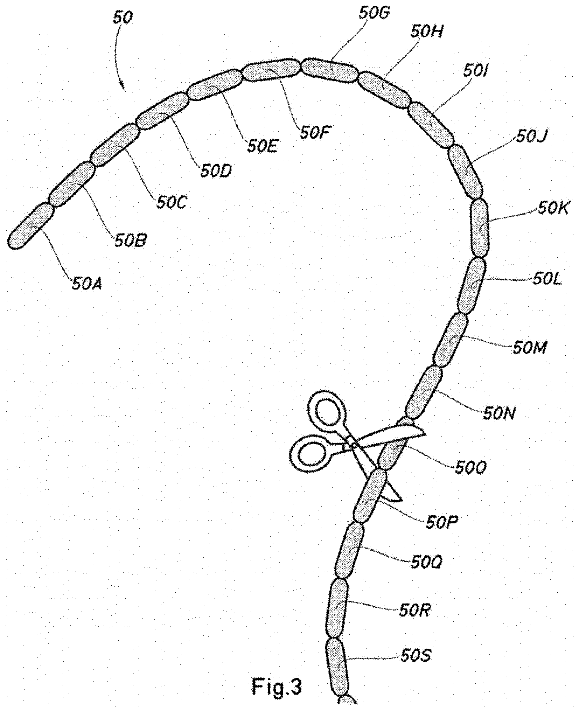

In the embodiment shown, the garment 1 comprises two trouser legs 10, 20 and each of them comprises a respective plurality of receptacles 100, 200 for accommodating a cold-accumulating material 50 (see Figures 3, 5 and 6). As can be seen, in this embodiment, the different receptacles 100, 200 are spaced substantially equidistant from each other, although in other embodiments said spacing may vary, among others, depending on the area of the body in which they are located and the effect that is intended to produce. The spacing between receptacles may also be due to aesthetic reasons.

As can be seen, in this embodiment the receptacles 100, 200 are arranged substantially perpendicular to the longitudinal axis of their respective trouser leg 10, 20, that is, substantially perpendicular to the longitudinal axis of the leg of the wearer of the garment 1. Similarly, in embodiments in which the garment covers the arms and/or the trunk of the wearer thereof, the receptacles and their respective cold-accumulating material may also be arranged substantially perpendicular to the longitudinal axis of the arms and/or the trunk.

In the embodiment shown, in an implied plan view, the receptacles 100, 200 and the respective cold-accumulating material 50 housed inside have a substantially circular or elliptical shape, so that they adapt to the shape of the extremity, in this case, the legs of the wearer of garment 1. As can be seen, by having an approximately circular section and a development around the leg or hip that is also approximately circular, the general shape of the receptacles turns out to be toric or of torus section. In other embodiments, the receptacles 100, 200 of substantially circular or elliptical shape may be replaced by one or more receptacles of substantially helical shape, said helix running along the entire extremity covered by the garment 1.

In the embodiment shown in Figures 1 and 2, each of the trouser legs 10, 20 of the garment 1 comprises a zone 110, 210, respectively, free of receptacle and cold-accumulating material. In that embodiment, the zones 110, 210 coincide approximately with the location of the knees of the wearer of the garment 1. In embodiments in which the garment is a T-shirt, arm sleeve or the like, said zone free of cold-accumulating material is preferably located in the zone of the wearer's elbow.

Although preferably the garments object of the present invention comprise the aforementioned areas free of cold-accumulating material, these being substantially coincident with the joints of the wearer's extremities, it is also possible to make garments that lack said areas, that is, garments that contain receptacles and respective cold-accumulating material distributed throughout the garment.

In the case of the embodiment shown, the garment 1 comprises two areas 30, 40 free of cold-accumulating material, both coinciding with the pubic area and the perianal or intergluteal area, respectively, of the wearer of the garment 1. The objective of the absence of cold-accumulating material in both areas is, among others, to increase the wearing comfort of the garment 1, since both the pubic and the perianal or intergluteal areas are especially sensitive to cold.

Figure 3 schematically shows an embodiment of a cold-accumulating material 50 according to the present invention. In said embodiment, the cold-accumulating material 50 is packaged in a plurality of concatenated capsules 50A, 50B, 50C, 50D, 50E, 50F, 50G, 50H, 501, 50J, 50K, 50L, 50M, 500, 50P, 50Q, 50R, 50S made of malleable plastic. This arrangement in concatenated capsules of the cold-accumulating material 50 has numerous advantages, especially with regard to the manufacture of the garment 1.

Due to the preferably anatomical nature of the garments object of the present invention, it is necessary that, in order to adapt to the shape of the body of the wearer thereof, each of the different receptacles 100, 200 and the cold-accumulating material 50 that they house have different lengths. This necessity to have strips of cold-accumulating material 50 of multiple lengths would entail a great stock problem, since the manufacture of garments according to the present invention would require having to stock a large number of strips of cold-accumulating material 50 of different lengths. However, this need for a large stock is solved by the fact that the cold-accumulating material 50 is packaged in concatenated capsules, thus allowing to cut the cold-accumulating material 50 into the necessary lengths from, for example, a single roll or coil of said material 50. In this way, it is possible to manufacture strips of cold-accumulating material 50 of different lengths that can adapt to different parts of the body and be used in garments of different sizes, etc., depending on the production needs and without necessitating a large stock volume.

Preferably, the cut is carried out in a joining zone between capsules formed by the packaging material thereof, that is, said joining zone does not comprise cold-accumulating material and allows the different concatenated capsules to be separated cleanly, that is, in other words, it allows the capsules to be separated without the need to cut a capsule, which would lead to its breakage and the loss of the cold-accumulating material contained therein.

In the embodiment shown, the cold-accumulating material 50 is a eutectic gel with a high calorific capacity and a high latent heat of fusion.

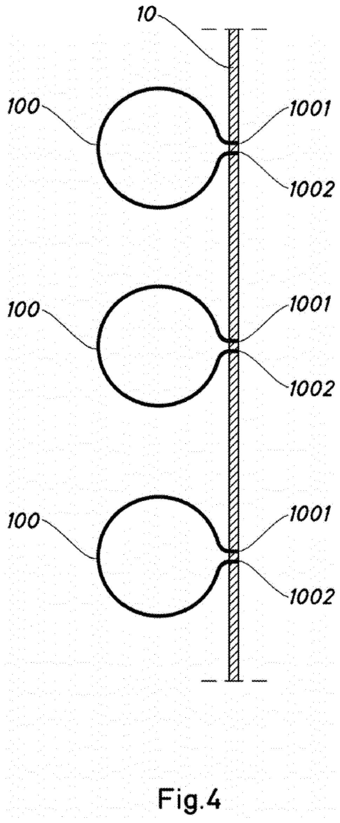

Figure 4 schematically illustrates a cross section of the receptacles for accommodating a cold-accumulating material of an embodiment of a garment for cryotherapy according to the present invention. In the embodiment shown, the receptacle 100 is made of the same material as the rest of the trouser leg 10 of the garment 1. The receptacle 100 is formed by a fold of the garment material. The figure shows the weave that forms the main face of the garment (fabric of the trouser leg 10) with a striped wall. This representation was used to cover various possible woven fabrics, in one, two or more layers, made of the same yarns or of different compositions and/or colour. Meanwhile, the walls of the receptacle 100 were represented by continuous lines exiting the fabric of the trouser leg 10 or garment, forming a fold and returning to the fabric. It should be understood that the walls of the receptacle may also be woven in different ways, and that the exit and return points of the fabric of the fold may vary, being able to exit from any point of the weave of the fabric of the trouser leg 10 or garment.

As can be seen, since it is formed by threads of the garment weave, both longitudinal ends 1001, 1002 of the receptacle 100 are attached to the fabric of the trouser leg 10. In the case shown, they leave a space between both joints, thus delimiting the receptacle 100 destined to house the cold-accumulating material 50. In other embodiments, both ends 1001, 1002 may be joined to or interlaced with each other by weaving techniques and, in turn, be attached to the fabric of the garment 1.

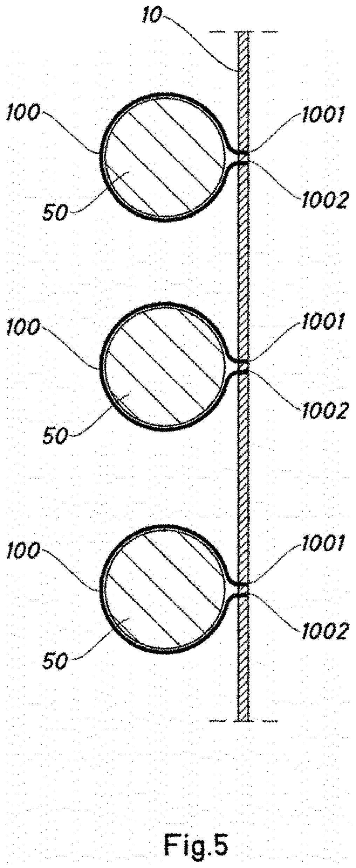

Figure 5 schematically illustrates a cross section of said receptacles housing their respective cold-accumulating material of an embodiment of a garment for cryotherapy according to the present invention. As can be seen, Figure 5 is very similar to Figure 4, except that cold-accumulating material 50 was added inside the receptacles 100.

In the example shown, the assembly formed by the receptacle 100 and its respective cold-accumulating material 50 have a section substantially circular in shape. This is due, among others, to the fact that the distance between the ends 1001, 1002 is quite small. However, in other embodiments, said spacing may be greater, so that the section formed by the receptacle 100, the material 50 and the trouser leg 10 has a U-shaped section, for example, thus increasing the contact surface between the cold-accumulating material 50 and the trouser leg 10, and therefore, increasing the transfer of heat between the cold-accumulating material 50 and the skin of the wearer of the garment 1. It is important to remember that due to the malleability of the cold-accumulating material 50 and the packaging that contains it, it can be adapted to some extent to the shape of the receptacle 100 that houses it.

In this embodiment, the fabric of the trouser leg 10 of the garment 1, in addition to giving body to the garment 1 and holding the receptacles 100, also serves to avoid direct contact between the cold-accumulating material 50 and the skin of the wearer of said garment, which could cause injuries and/or cold burns to said wearer. Although in the embodiment shown in Figures 4 and 5, the fabric of the trouser leg 10 has only one layer, in other embodiments the fabric may have more than one layer.

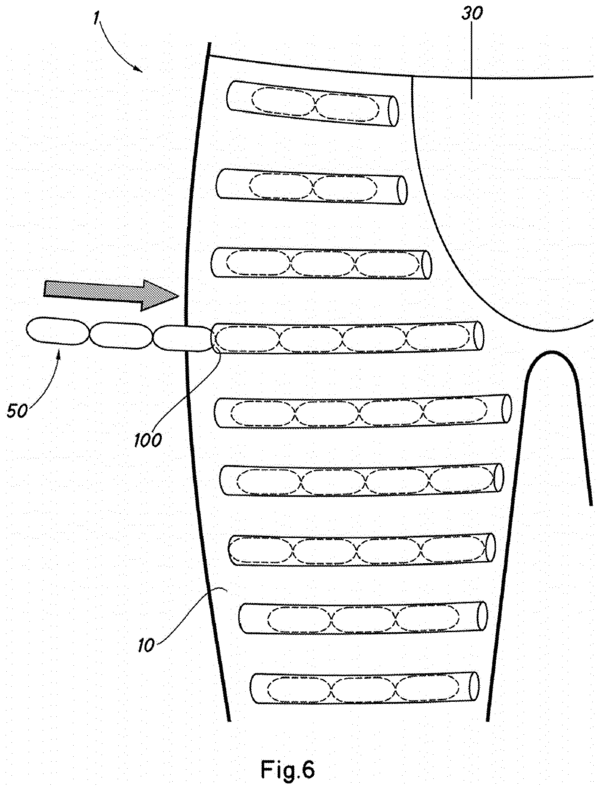

Figure 6 schematically shows the introduction of the cold-accumulating material into its respective receptacle. As can be seen, the lengths of the cold-accumulating material 50 and its respective receptacle 100 are different depending on the part of the garment in which they are located. For the introduction of the cold-accumulating material 50 into its respective receptacle 100, said cold-accumulating material 50 is first cut into the required length (see Figure 3). Since the material of the receptacle 100, in the same way as that of the rest of the garment, is preferably elastic, it allows the cold-accumulating material 50 to be inserted inside it and, once inserted, to compress and hold it in place. To minimise the risk that the cold-accumulating material 50 will come out of its respective receptacle 100, all of its ends may be closed up. Said closure is preferably made by sewing, although other types of closure are also possible.

Although an embodiment of a garment according to the present invention was shown and described in the figures, in which said garment is in the form of tights, it should be understood that what was explained is equally applicable to embodiments in which the garment is a T-shirt, an arm sleeve, a calf sleeve, etc.

While the invention was described and represented on the basis of several representative examples, it should be understood that such embodiments are in no way limiting to the present invention, so that any variations that may be included directly or otherwise by way of equivalence in the content of the appended claims, should be considered included in the scope of the present invention.

## Claims

1. Garment for cryotherapy comprising several receptacles to house a cold-accumulating material, **characterised in that** each of said receptacles is formed by a closed fold of garment fabric, each one of said receptacles presenting two end openings for the introduction of a container of cold-accumulating material.

2. Garment, according to the preceding claim, **characterised in that** the receptacles have an elongated shape.

3. Garment, according to the preceding claim, **characterised in that** the receptacles have a toric or torus-section shape.

4. Garment for cryotherapy, according to claim 2 or 3, **characterised in that** said fold is closed longitudinally along one or two lines of closure.

5. Garment for cryotherapy, according to any of the preceding claims, **characterised in that** said fold is closed by weaving techniques.

6. Garment for cryotherapy, according to the preceding claim, **characterised in that** the garment comprises containers of cold-accumulating material formed by concatenated malleable plastic capsules capable of being introduced into the receptacles through one of the aforementioned end openings of a respective receptacle.

7. Garment, according to the preceding claim, **characterised in that** there is a container of cold-accumulating material composed of several concatenated capsules inside each receptacle.

8. Garment according to claim 6 or 7, **characterised in that** the concatenated capsules are spaced at regular intervals by pinching zones.

9. Garment according to claim 8, **characterised in that** the capsules are joined together by 1-centimetre pinching zones arranged every 4 centimetres.

10. Garment according to any of the preceding claims, **characterised in that** it is compressive.

11. Garment, according to any of the preceding claims, **characterised in that** said garment fabric comprises an elastomer.

12. Garment for cryotherapy, according to any of the preceding claims, **characterised in that** said cold-accumulating material is a gel.

13. Garment, according to any of the preceding claims, **characterised in that** it comprises at least two receptacles for each end to be covered by the garment.

14. Garment for cryotherapy, according to the preceding claim, **characterised in that** said receptacle is arranged substantially perpendicular to the longitudinal axis of its respective extremity.

15. Garment for cryotherapy, according to any of the preceding claims, **characterised in that** it is a pair of tights.

16. Garment, for cryotherapy, according to the preceding claim, **characterised in that** the receptacles cover from the iliac crest to the ankles, with the exception of areas corresponding to the inner face of the legs, genital area and intergluteal cleft.

17. Garment for cryotherapy, according to any of claims 1 to 14, **characterised in that** it is a T-shirt.

18. Garment for cryotherapy, according to any of claims 1 to 14, **characterised in that** it is an arm sleeve.

19. Garment for cryotherapy, according to any of claims 1 to 14, **characterised in that** it is a calf sleeve.
